# EUROPEAN PATENT SPECIFICATION

(11) **EP 1 807 130 B1**
(45) Date of publication and mention of the grant of the patent: **05.05.2010**
(21) Application number: 05821057.6
(22) Date of filing: 01.11.2005
(51) Int. Cl.: A61L 31/12, A61L 31/16, A61L 29/16, A61L 29/12

(54) **MEDICAL DEVICE FOR DELIVERING THERAPEUTIC AGENTS OVER DIFFERENT TIME PERIODS**
MEDIZINISCHE VORRICHTUNG ZUR VERABREICHUNG THERAPEUTISCHER WIRKSTOFFE ÜBER UNTERSCHIEDLICHE ZEITABSCHNITTE
DISPOSITIF MEDICAL D'ADMINISTRATION D'AGENTS THERAPEUTIQUES SUR DIFFERENTES PERIODES

(30) Priority: 04.11.2004 US 982355
(43) Date of publication of application: 18.07.2007
(73) Proprietor: Boston Scientific Limited, Christ Church (BB)
(72) Inventor: STENZEL, Eric, B., Galway (IE)
(74) Representative: Peterreins, Frank
(86) International application number: PCT/US2005/039592
(87) International publication number: WO 2006/052575

(56) References cited:
- WO-A-03/035131
- WO-A-2004/028407
- WO-A-2004/037443
- US-A1- 2004 215 313
- US-B1- 6 419 692
- US-B1- 6 783 793

## Description

### 1. FIELD OF THE INVENTION

The present invention relates generally to medical devices, such as stents, for delivering a therapeutic agent to a desired location within the body of a patient, such as a body lumen. More particularly, the medical device has a surface that is coated with at least two different coating regions that deliver a therapeutic agent to a patient over different time periods.

### 2. BACKGROUND OF THE INVENTION

A variety of medical conditions are commonly treated by introducing an insertable or implantable medical device into the body. In many instances, the medical device is coated with a material, such as a polymer, which is capable of releasing a therapeutic agent. For example, various types of drug-coated stents have been used for localized delivery of drugs to a body lumen. *See, e.g.,* U.S. Patent Nos. 6,099,562, 6,153,252 and 6,156,373.
WO2004037443 and WO2004/028407 separately describes a medical device for delivering a therapeutic agent to a body tissue comprising a medical device having a surface, a first coating region comprising a therapeutic agent, a second coating region comprising a therapeutic agent disposed on a second portion of the medical surface.
WO03/035131 describes a medical device for delivering a therapeutic agent to a body tissue comprising a medical device having a surface, a first coating region comprising a therapeutic agent, a second coating region comprising a therapeutic agent disposed on a second portion of the medical surface. It is also mentioned that a deposition of a diffusion layer on selected coated regions is possible to get selected release.
US2004/215313 describes a medical device for delivering a therapeutic agent to a body tissue comprising a medical device having a surface, a first coating region comprising a therapeutic agent, a second coating region comprising a therapeutic agent disposed on a second portion of the medical surface. Moreover, in document D3, a third coating layer which can also includes drugs or therapeutic agents. Different therapies and delivery timing can be achieved by selection of different materials and therapeutics agents for the different coating layers.
US6419692 describes a medical device for delivering a therapeutic agent to a body tissue comprising a medical device having a surface, a first coating region comprising a therapeutic agent, a second coating region comprising a therapeutic agent disposed on a second portion of the medical surface.

Generally, the current coated medical devices release the therapeutic agent over a single time period. However, in some applications, it may be desirable to have the therapeutic agent released or delivered from the medical device coating over several different time periods. For instance, it may be desirable to have some of the therapeutic agent begin to release soon after the medical device is implanted and have some of the therapeutic agent begin to release at subsequent time(s). Therefore, there is a need for a medical device having a coating comprising a therapeutic agent in which the therapeutic agent is released over more than one time period, *i.e*., over different time periods.

### 3. SUMMARY OF THE INVENTION

The embodiments of the present invention related to medical devices, such as stents, that have a surface coated with at least two coating regions comprising a therapeutic agent as described in claim 1. The therapeutic agent begins to release from the coating regions at different times, *i.e*.. the therapeutic agent from each coating region is released over different time periods.

In one embodiment, the medical device for delivering a therapeutic agent to a body tissue of a patient comprises a medical device having a surface. The medical device also comprises (a) a first coating region disposed on a first portion of the medical device surface, in which the first coating region comprises a first coating layer comprising a first therapeutic agent and (b) a second coating region disposed on a second portion of the medical device surface. The second coating region comprises a second coating layer comprising a second therapeutic agent; and at least a first additional coating layer disposed over the second coating layer. The first additional coating layer comprises a first biodegradable material and is capable of preventing the second therapeutic agent of the second coating layer from beginning to release from the second coating layer at the same time as the first therapeutic agent of the first coating layer begins to release from the first coating layer. The first coating region is capable of releasing the first therapeutic agent before the second coating region begins to release the second therapeutic agent. In certain embodiments, the second therapeutic agent begins to release before the release of the first therapeutic agent is completed. In other embodiments, the second therapeutic agent begins to release after the release of the first therapeutic agent is completed.

In certain embodiments, the first and second therapeutic agents can be the same. The first coating layer is not covered by any other coating layer. In addition, the first coating layer and the second coating layer can be contiguous. Moreover, the first additional coating layer can be disposed directly over the second coating layer or the first additional coating layer can be disposed indirectly over the second coating layer. In certain embodiments, the medical device can further comprise an intermediate coating layer disposed between the second coating layer and the first additional coating layer. The intermediate coating layer can comprise a biodegradable material and/or a third therapeutic agent. In some embodiments, the first coating layer further comprises a polymeric material and/or the second coating layer further comprises a polymeric material.
In some embodiments, the first therapeutic agent can comprise an anti-thrombogenic agent, an anti-angiogenesis agent, an anti-proliferative agent, a growth factor, or a radiochemical. The anti-proliferative agent comprises paclitaxel, a paclitaxel analogue or a paclitaxel derivative.

In certain embodiments, the medical device can be a stent having a tubular sidewall in which the first portion of the medical device surface lies along a circumference of the tubular sidewall. In some embodiments, the medical device can be a stent having a tubular sidewall in which the first portion of the medical device surface lies along a longitudinal axis of the tubular sidewall.

In some embodiments, the medical device further comprises a third coating region disposed over a third portion of the medical device surface. The third coating region comprises a third coating layer comprising a third therapeutic agent; and at least a second additional coating layer disposed over the third coating layer. The second additional coating layer comprises a second biodegradable material. In some embodiments, the first, second and third therapeutic agent can be the same. In certain embodiments, the second additional coating layer can be disposed directly over the third coating layer or the second additional coating layer can be disposed indirectly over the third coating layer. Also, the medical device of claim can further comprise an intermediate coating layer disposed between the second coating layer and the first additional coating layer. The intermediate coating layer can comprise a biodegradable material and/or a fourth therapeutic agent. In certain embodiments, the first coating layer, is contiguous with at least the second coating layer or the third coating layer. Also, in some embodiments, the medical device further comprises at least a third additional coating layer disposed over the second additional coating layer. The third additional coating layer can comprise a third biodegradable material. In certain embodiments, the second additional coating layer further comprises a biologically active material.

In some embodiments, the first and second biodegradable materials can degrade at the same rate or the first and second biodegradable materials can degrade at different rates. In certain embodiments, the first additional coating layer can have a first thickness and the second additional coating layer can have a second thickness in which the first and second thicknesses are not the same. In other embodiments, the first and second thicknesses are the same. In some embodiments, the medical device is a stent having a tubular sidewall having an inner surface and an outer surface in which the first coating region and second coating region are disposed on the outer surface.

Moreover, in certain embodiments, the medical device further comprises a third coating region disposed on the inner surface. The third coating region comprises a third coating layer comprising a third therapeutic agent; and at least a second additional coating layer disposed over the third coating layer. The second additional coating layer can comprise a second biodegradable material.

In addition, in some embodiments, the medical device is a stent for delivering a therapeutic agent to patient which comprises a surface. The stent further comprises a first coating region disposed on a first portion of the surface. The first coating region comprises a first coating layer comprising a first therapeutic agent and a first polymeric material. Also, the stent comprises a second coating region disposed on a second portion of the medical device surface. The second coating region comprises a second coating layer comprising a second therapeutic agent and a second polymeric material; and at least an additional coating layer disposed over the second coating layer. The additional coating layer comprises a first biodegradable material. Also, the additional coating layer is capable of preventing the second therapeutic agent of the second coating layer from beginning to release from the second coating layer at the same time as the first therapeutic agent of the first coating layer begins to release from the first coating layer. The first coating region is capable of releasing the first therapeutic agent before the second coating region begins to release the second therapeutic agent. In certain embodiments, the second therapeutic agent begins to release before the release of the first therapeutic agent is completed. In other embodiments, the second therapeutic agent begins to release after the release of the first therapeutic agent is completed. Furthermore, the first coating layer and the second coating layer are contiguous and the additional coating layer is disposed directly over the second coating layer.

Moreover, in certain embodiments, the medical device is a stent for delivering a therapeutic agent to a patient comprising a tubular sidewall having an outer surface and an inner surface. The stent further comprises a first coating region disposed on a first portion of the outer surface, wherein the first coating region comprises a first coating layer comprising a first therapeutic agent and a polymeric material. The stent also comprises a second coating region disposed on a second portion of the outer surface. The second coating region comprises a second coating layer comprising a second therapeutic agent and the polymeric material; and at least a first additional coating layer disposed over the second coating layer. The first additional coating layer comprises a first biodegradable material and the first additional coating layer is capable of preventing the second therapeutic agent of the second coating layer from beginning to release from the second coating layer at the same time as the first therapeutic agent of the first coating layer begins to release from the first coating layer. The first coating region is capable of releasing the first therapeutic agent before the second coating region begins to release the second therapeutic agent. In certain embodiments, the second therapeutic agent begins to release before the release of the first therapeutic agent is completed. In other embodiments, the second therapeutic agent begins to release after the release of the first therapeutic agent is completed. The stent further comprises a third coating region disposed on a portion of the inner surface. The third coating region comprises a third coating layer comprising a third therapeutic agent and the polymeric material; as well as at least a second additional coating layer disposed over the third coating layer. The second additional coating layer comprises a second biodegradable material. Also, the first coating layer, and the second coating layer are contiguous and the first additional coating layer is disposed directly over the second coating layer. In some embodiments, the medical device comprises at least a third additional coating layer disposed over the second additional coating layer. In certain embodiments, third additional coating layer comprises a third biodegradable material. In addition to using biodegradable materials as the additional coating layers, bioabsorbable materials may also be used.

### 4. BRIEF DESCRIPTION OF THE DRAWINGS

**Fig. 1** shows a side view of a stent suitable for use in the present invention.

**Figs. 2A-2E** are cross-sectional views of embodiments of a coated medical device surface in accordance with the present invention.

**Figs. 3A-3C** are cross-sectional views of other embodiments of a coated medical device surface of the present invention.

**Fig. 4** is a cross-sectional view of an additional embodiment of a coated medical device surface of the present invention.

**Figs. 5A-5B** show a stent having coating regions disposed along the longitudinal axis of the stent.

**Figs. 6A-6B** show a stent having coating region disposed along the circumference of the stent.

### 5. DETAILED DESCRIPTION

The medical devices of the present invention comprise a medical device as described in claim 1. Suitable medical devices include, but are not limited to, catheters, guide wires, balloons, filters (*e.g*., vena cava filters), stents, stent grafts, vascular grafts, intraluminal paving systems, implants and other devices. In certain embodiments, the medical devices are implanted or otherwise utilized in body lumina and organs such as the coronary vasculature, cranial vasculature, esophagus, trachea, colon, biliary tract, urinary tract, prostate, brain, and the like.

The filters that can be used in accordance with the present invention include, for example, thrombus filters that can be placed at a selected location within the vascular system and removed when no longer required. A preferred location for placement of these filters is the vena cava. Filters placed in the vascular system can intercept blood clots that may otherwise travel to the lungs and result in a pulmonary embolism, a life-threatening emergency that has become increasingly common. Further examples of filters that may be used in accordance with present invention include, e.g., those described in International Application No. WO 96/17634 and International Application No. WO 96/12448.

The grafts, including stent grafts, that may be used in accordance with the present invention include synthetic vascular grafts that can be used for replacement of blood vessels in part or in whole. A typical vascular graft is a synthetic tube with each end thereof sutured to the remaining ends of a blood vessel from which a diseased or otherwise damaged portion has been removed. In a typical stent graft, each end of the synthetic tube portion includes a stent that is affixed to each of the remaining ends of a blood vessel from which a diseased or otherwise damaged portion has been removed. Examples of other suitable grafts are described in U.S. Pat. Nos. 5,509,931, 5,527,353, and 5,556,426.

Examples of suitable stents include without limitation such as those described in U. S. Patent No. 6,478,816 to Kveen et al.*,* U.S. Patent Nos. 4,655,771 and 4,954,126 issued to Wallsten and 5,061,275 issued to Wallsten et al. as well as U.S. Patent No. 5,449,373 issued to Pinchasik et al.

The medical devices suitable for the invention may be fabricated from metallic, ceramic, polymeric materials, non-polymeric materials or combinations thereof. The material may be porous or nonporous. Porous structural elements can be microporous, nanoporous or mesoporous. Preferred materials are metallic. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. The components may also include parts made from other metals such as, for example, gold, platinum, or tungsten. The medical device may also include non-alloy combinations such as plated metals including but not limited to, for example, gold-plated or tantalum-plated stainless steel. Metallic materials also include clad composite filaments, such as those disclosed in WO 94/16646.

Suitable ceramic materials include, but are not limited to, oxides, carbides, or nitrides of the transition elements such as titaniumoxides, hafnium oxides, iridiumoxides, chromium oxides, aluminum oxides, and zirconiumoxides. Silicon based materials, such as silica, may also be used.

The polymer(s) useful for forming the medical devices should be ones that are biocompatible and avoid irritation to body tissue. The polymers can be either biostable or bioabsorbable. Suitable polymeric materials include without limitation polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, polystyrene, isobutylene, cellulose, collagens, and chitins.

Other polymers that are useful include, without limitation, dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) co-polymer, polylactic acid, poly(y-caprolactone), poly(γ -hydroxybutyrate), polydioxanone, poly(γ -ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, *e.g*., RGD, in which the polymers retain their structural integrity while allowing for attachment of cells and molecules, such as proteins, nucleic acids, and the like. Furthermore, although the invention can be practiced by using a single type of polymer to form the medical device, various combinations of polymers can be employed. The appropriate mixture of polymers can be coordinated to produce desired effects when incorporated into a medical device.

Medical devices may be made with non-polymeric materials. Examples of useful non-polymeric materials include sterols such as cholesterol, stigmasterol, β-sitosterol, and estradiol; cholesteryl esters such as cholesteryl stearate; C₁₂-C₂₄ fatty acids such as lauric acid, myristic acid, palmitic acid, stearic acid, arachidic acid, behenic acid, and lignoceric acid; C₁₈-C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; C₁₆-C₁₈ fatty alcohols such as cetyl alcohol, myristyl alcohol, stearyl alcohol, and cetostearyl alcohol; esters of fatty alcohols and fatty acids such as cetyl palmitate and cetearyl palmitate; anhydrides of fatty acids such as stearic anhydride; phospholipids including phosphatidylcholine (lecithin), phosphatidylserine, phosphatidylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymeric materials include cholesterol, glyceryl monostearate, glycerol tristearate, stearic acid, stearic anhydride, glyceryl monooleate, glyceryl monolinoleate, acetylated monoglycerides, fibrin, lactose, cellulose, polyvinylpyrrolidone, crospovidone, polyethylene glycol, and Enduragit^{™}.

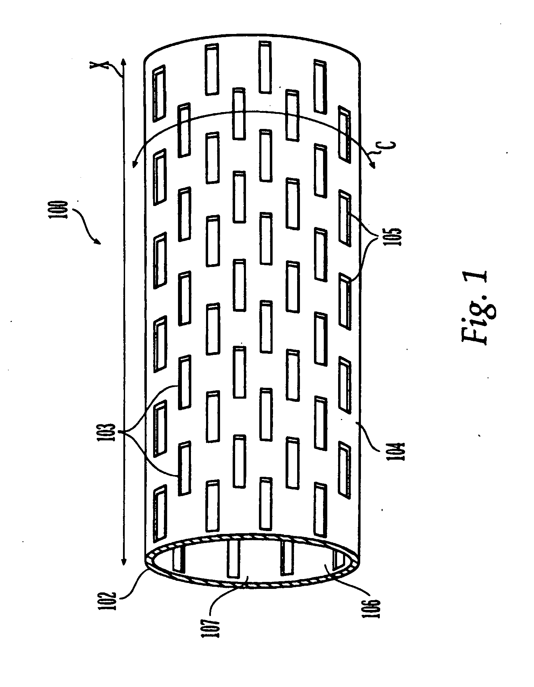

**Fig. 1** shows an example of a stent **100** that can be used in the present invention. As discussed above, medical devices in addition to stents can be used in the present invention. The stent **100** comprises a tubular sidewall **102** comprising a plurality of struts **103.** Also the stent **100** tubular sidewall **102** has an outer surface **104** having a plurality of openings **105** therein, an inner surface **106** having a plurality of openings **105** therein, and a flow path **107** that runs along the longitudinal axis **X** of the stent **100.** The outer surface **104** is the surface facing away from the flow path **107** and the inner surface **106** is the surface facing the flow path **107.** The stent **100** also has a circumference **C.**

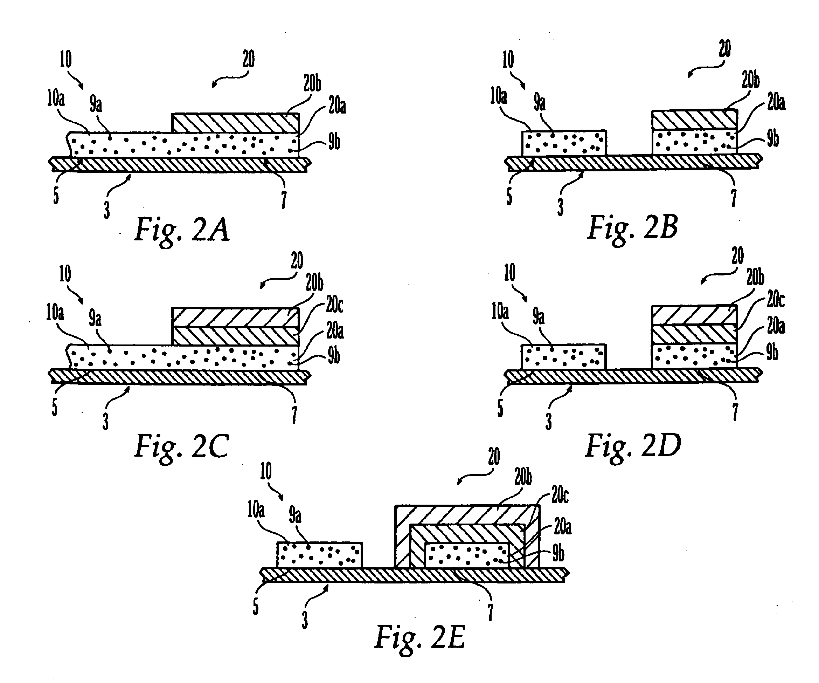

**Fig. 2A** shows an embodiment of the present invention. More specifically, this figure shows a cross-sectional view of a surface **3** of a medical device that is coated with a first coating region **10** and a second coating region **20.** The first coating region **10** is disposed on a first portion of the surface **5** and the second coating region **20** is disposed on a second portion of the surface **7.**

The first coating region **10** comprises a first coating layer **10a,** which comprises a first therapeutic agent **9a.** This first coating layer **10a** can also comprise a polymeric material, such as those described below. In some embodiments, the polymeric material can incorporate the therapeutic agent. Although the first coating layer **10a** is shown as being directly disposed on the first portion of the surface **5,** *i.e.* in contact with the first portion of the surface **5,** the first coating layer **10a** can be indirectly disposed on the first portion of the surface **5.** In such instance, an intermediate coating layer may be disposed between the first portion of the surface **5** and the first coating layer **10a.** Also, as shown in **Fig. 2A****,** in some embodiments, the first coating layer **10a** not be covered by any other coating layer.

The second coating region **20** comprises a second coating layer **20a** and a first additional coating layer **20b.** The second coating layer **20a** comprises a second therapeutic agent **9b,** which could be the same as or different than the first therapeutic agent **9a.** Also, the second coating layer **20a** can include other therapeutic agents in addition to the second therapeutic agent **9b.** Also, like the first coating layer **10a,** the second coating layer **20a** can also comprise a polymeric material. In some embodiments, the polymeric material can incorporate the therapeutic agent. Although the second coating layer **20a** is shown as being directly disposed on the second portion of the surface **7,** *i.e.* in contact with the second portion of the surface **7,** the second coating layer **20a** can be indirectly disposed on the second portion of the surface **7.** For instance, an intermediate coating layer may be disposed between the second portion of the surface **7** and the second coating layer **20a.**

The first additional coating layer **20b** is disposed over the second coating layer **20a.** The first additional coating layer **20b** comprises a biodegradable material, such as a biodegradable polymer. Although not shown in **Fig. 2A****,** the second coating region **20** can include other additional coating layers, such as a second additional coating layer disposed over the first additional coating layer **20b.** Also, although **Fig. 2a** shows the first additional coating layer **20b** covering the entire top surface of the second coating layer **20a,** the first additional coating layer **20b** or any other overlying layer may cover only a portion of the second coating layer **20a** or other underlying coating layer.

The inclusion of the first additional coating layer **20b** can prevent the therapeutic agent **9b** of the second coating layer **20a** from beginning to release from the second coating layer **20a** at the same time that the therapeutic agent **9a** of the first coating layer **10a** begins to release from the first coating layer **10a.** Since the second coating layer **20a** is covered by the first additional coating layer **20b,** in certain embodiments the second therapeutic agent **9b** of the second coating layer **20a** will not begin releasing from the second coating layer **20a** generally until a significant part of the biodegradable first additional coating layer **20b** has dissociated, *i.e*. degraded or absorbed. In contrast, because the first coating layer **10a** is not covered by any additional coating layer, the first therapeutic agent **9a** of the first coating layer **10a** can begin to release from the first coating layer **10a** without waiting for the degradation of an additional coating layer. Therefore, the inclusion of the first additional coating layer **20b** can cause the therapeutic agents of the first and second coating layers, **10a** and **20a,** to be released over different time periods.

Also, in the embodiment shown in **Fig. 2A****,** the first coating layer **10a** and second coating layer **20a** are contiguous, *i.e.* in contact with each other. In such an embodiment the first coating region **10** and second coating region **20** are in contact with each other. In other embodiments, such as that shown in **Fig. 2B****,** the first and second coating regions can be spaced apart. Also, it should be noted that while **Figs. 2A-2B** show only two coating regions, the surface **3** can be coated with additional coating regions.

**Figs. 2C-2D** show embodiments of the coated medical device surface wherein the second coating region **20** comprises an intermediate layer **20c** disposed between the second coating layer **20a** and the first additional coating layer **20b.** In these embodiments, the first additional coating layer **20b** is indirectly disposed on or not in contact with the second coating layer **20a.** In contrast, the first additional coating layer **20b** is directly disposed on the second coating layer **20a** in the embodiments of **Figs. 2A-2B****.** Preferably, the intermediate layer **20c** comprises a biodegradable material that is the same or different as the biodegradable material of the first additional coating layer **20b.** Also, the biodegradable material of the intermediate layer **20c** can degrade at the same or a different rate than the biodegradable material of the first additional layer **20b.** In some embodiments, the intermediate layer **20c** can include a therapeutic agent.

In certain embodiments, such as that shown in **Fig. 2E****,** the first additional coating layer **20b,** or any other overlying layer, may not only cover the top surface of the second coating layer **20a** or other underlying layer, such as an intermediate layer **20c,** but also the side surfaces of the underlying layer. In this figure, the intermediate layer **20c** covers the top and side surfaces of the second coating layer **20a.** The first additional coating layer 20b covers the top and side surfaces of the intermediate layer **20c.**

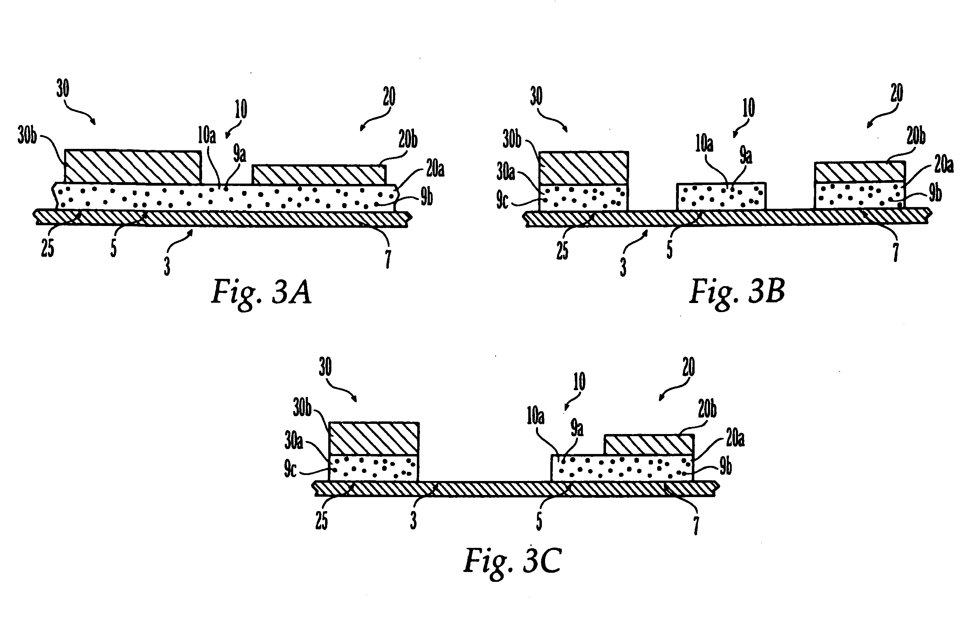

**Figs. 3A-3C** show embodiments where the medical device surface **3** is coated with a third coating region **30** that is disposed over a third portion of the surface **25.** The third coating region **30,** comprises a third coating layer **30a** and a second additional coating layer **30b.**

The third coating layer **30a** comprises a third therapeutic agent **9c** that can be the same or different as the therapeutic agents **9a** and **9b** of the first and second coating layers, **10a** and **20a** respectively. Also, the third coating layer **30a** can also include therapeutic agents in addition to the third therapeutic agent. Also, like the first and second coating layers **10a, 20a** the third coating layer **30a** can also comprise a polymeric material. In some embodiments, the polymeric material can incorporate the therapeutic agent. Although the third coating layer **20a** is shown as being directly disposed on the third portion of the surface **25,** *i.e.* in contact with the third portion of the surface **25** the third coating layer **30a** can be indirectly disposed on the third portion of the surface **25.** For instance, an intermediate coating layer may be disposed between the third portion of the surface **25** and the third coating layer **30a.**

The second additional coating layer **30b** is disposed over the third coating layer **30a.** The second additional coating layer **30b** comprises a biodegradable material, such as a biodegradable polymer. Although not shown in **Figs. 3A-3C****,** the third coating region **30** can include other additional coating layers, such as a third additional coating layer disposed over the second additional coating layer **30b.** Moreover, the first and second additional coating layers, **20b** and **30b** can have the same or different thicknesses.

Like the first additional coating layer **20b,** the inclusion of the second additional coating layer **30b** affects the time when the therapeutic agent of its underlying coating layer(s) begins to release from the underlying coating layers, *e.g*., third coating layer **30a.** Therefore, inclusion of this second additional coating layer **30b** can result in a third coating region **30** that releases its therapeutic agent over a time period that is different than the time period(s) over when at least one other coating region releases its therapeutic agent.

In the embodiment shown in **Fig. 3A****,** the first coating layer **10a** is contiguous with the second coating layer **20a** which is contiguous with the third coating layer **30a.** In other embodiments, such as that shown in **Fig. 3B****,** the first, second and third coating regions are spaced apart. Alternatively, as shown in **Fig. 3C****,** the first and second coating regions **10, 20,** can be contiguous while the third coating region **30** is spaced apart from the first and second coating regions **10, 20.** Also, it should be noted that while **Figs. 3A-3C** show only three coating regions, the surface **3** can be coated with additional coating regions.

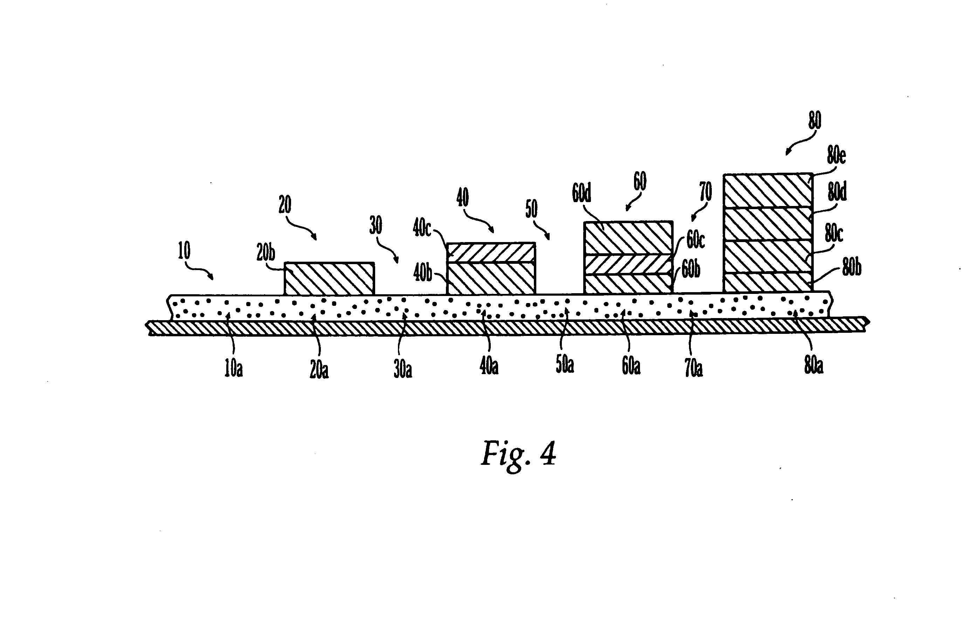

**Fig. 4** shows an embodiment where the medical device surface is coated with 8 coating regions, **10, 20, 30, 40, 50, 60, 70** and **80.** The first coating region **10,** the third coating region **30,** the fifth coating region **50** and the seventh coating region **70** each comprise a single coating layer, **10a, 30a, 50a** and **70a** respectively. These coating layers may comprise a therapeutic agent, which can be the same or different in each of the coating layers. In other embodiments, these coating layers may be free of any therapeutic agent. The coating layers **10a, 30a, 50a,** and **70a,** are not covered by any other coating layer.

The second, fourth, sixth and eighth coating regions **20, 40, 60** and **80** include at least one biodegradable coating layer overlying a coating layer **20a, 40a, 60a** and **80a** that comprises a therapeutic agent. The second coating region **20** comprises one additional coating layer **20b** that overlies coating layer **20a,** which includes a therapeutic agent. Preferably, the biodegradable additional coating layer **20b** comprises a biodegradable polymeric material.

The fourth coating region **40** comprises two additional biodegradable coating layers **40b, 40c** that overlie coating layer **40a,** which includes a therapeutic agent. Preferably, the biodegradable additional coating layers **40b, 40c** comprise a biodegradable polymeric material. Alternatively, either or both additional coating layers **40b, 40c** can comprise a therapeutic agent and/or a biodegradable polymeric material.

The sixth coating region **60** comprises three additional biodegradable coating layers **60b, 60c** and **60d** that overlie coating layer **60a,** which includes a therapeutic agent. Preferably, the biodegradable additional coating layers **60b, 60c** and **60d** comprise a biodegradable polymeric material. Alternatively, any of the additional coating layers **60b, 60c** and **60d** can comprise a therapeutic agent and/or a biodegradable polymeric material.

The eighth coating region **80** comprises four additional biodegradable coating layers **80b, 80c, 80d** and **80e** that overlie coating layer **80a,** which includes a therapeutic agent. Preferably, the biodegradable additional coating layers **80b, 80c, 80d** and **80e** comprise a biodegradable polymeric material. Alternatively, any of the additional coating layers **80b, 80c, 80d** and **80e** can comprise a therapeutic agent and/or a biodegradable polymeric material. For example, additional coating layers **80b** and **80c** may contain therapeutic agents and a biodegradable polymeric material while additional coating layers **80d** and **80e** contain a biodegradable polymeric material without any therapeutic agent.

Since the coating regions shown in **Fig. 4****,** comprise different numbers of additional coating layers comprising various coating materials, the time when the therapeutic agent of the underlying coating layers **10a, 20a, 30a, 40a, 50a, 60a, 70a** and **80a** begins to be released from the each underlying coating layer can vary. Therefore, in this embodiment the time periods when the therapeutic agent(s) of the underlying coating layers **10a, 20a, 30a, 40a, 50a, 60a, 70a** and **80a** are release may generally vary from coating region to coating region.

In some embodiments, the coating region can begin to deliver the therapeutic agent immediately after the medical device is implanted. In specific embodiments, the coating region can begin to deliver the therapeutic agent about 10 to 60 minutes, 1-3 hours, 3-5 hours, 5-12 hours, 12-24 hours, 1-2 days, 2-7 days, 1-2 weeks, 2-4 weeks, 1-3 months, 3-6 months, 6-9 months, 9-12 months, 1-2 years, 2-4 years, 4-6 years, or 6-10 years after implantation of the medical device. In certain embodiments, the coating region can release the therapeutic agent for a period of at least about 10-60 minutes, 1-3 hours, 3-5 hours, 5-12 hours, 12-24 hours, 1-2 days, 2-7 days, 1-2 weeks, 2-4 weeks, 1-3 months, 3-6 months, 6-9 months, 9-12 months, 1-2 years, 2-4 years, 4-6 years, or 6-10 years. However, depending on the properties of the biodegradable/bioabsorbable layers, the actual therapeutic release time may be longer or shorter.

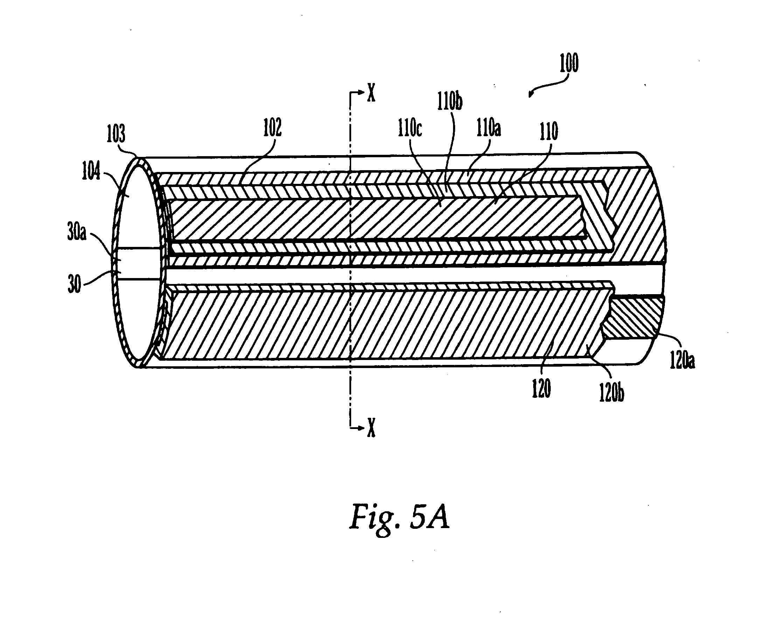

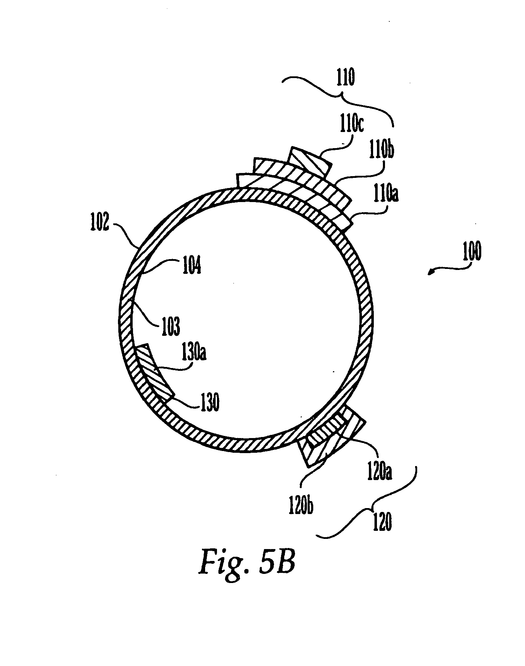

The coating region can be disposed on the surface of the medical device in any desired configuration or shape, *e.g*. circle, rectangle, etc. or may even be disposed in a pattern. For example, when the medical device is a stent, the coating regions can be disposed along the longitudinal axis of the stent as shown in **Fig. 5A****.** **Fig. 5B** shows a cross-sectional view of the stent of **Fig. 5A** along line X-X. In this embodiment, there are two coating regions **110** and **120** disposed on the outer surface of the stent and a third coating region **130** disposed on the inner surface of the stent. In some embodiments the coating regions are disposed on either the outer surface and/or the inner surface.

**Fig. 5A** shows a stent **100** having two coating regions, a first coating region **110** and a second coating region **120,** disposed on the outer surface **102** of its sidewall **103** as well as a third coating region **130** disposed on the inner **104** surface of the sidewall **103.** Although not shown, the side wall **103** can include a plurality of openings that extend through the outer surface **102** and inner surface **104.** As shown in the cut-away section of the first coating region **110** in **Fig. 5A****,** this coating region comprises three coating layers: a first coating layer **110a,** a first additional coating layer **110b** and a second additional coating layer **110c.** In this embodiment the second additional coating layer **110c** does not cover the entire first additional coating layer **110b,** which in turn does not cover the entire first coating layer **110a.** **Fig. 5B** shows a cross-sectional view of this coating region. In other embodiments this region can include further coating layers.

The second coating region **120** comprises a second coating layer **120a** and a third additional layer **120b.** In this embodiment, as shown in the cut-away section of **Fig. 5A** and the cross-section view of **Fig. 5B****,** the overlying third additional coating layer **120b** covers the entire underlying second coating layer **120a.** In certain embodiments, the overlying coating layers do not have to cover the entire underlying coating layer, *e.g*., cover just the top surface of the underlying layer. The third coating region **130** comprises a single third coating layer **130a** which is not covered by another coating layer. In some embodiments the third coating region **130** can include additional coating layers. Although the coating regions are shown as extending the entire length of the stent, in some embodiments, some or all of the coating regions may only extend a part of the length of the stent. Also, in some embodiments there may be more or fewer coating regions, having various numbers of layers.

In some embodiments, the first, second and/or third coating layers, **110a, 120a** and **130a** comprise a therapeutic agent. In certain embodiments, the first, second and/or third additional coating layers, **110b, 120b** and **110a** comprise a biodegradable material and/or a therapeutic agent.

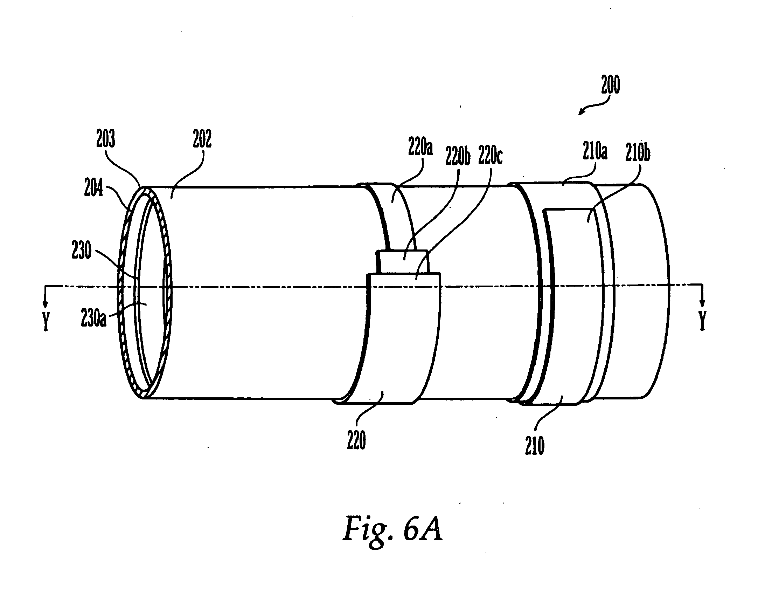

Alternatively, the coating regions can be disposed along the circumference of the stent as shown as **Figs. 6A-6B****.** As shown in **Figs. 6A-6B****,** in this embodiment, there are three coating regions **210, 220** and **230.** The first coating region **210** and second coating region **220** are disposed on the outer surface **202** of the sidewall **203** of the stent **200.** The third coating region **230** is disposed on the inner surface **204** of the sidewall **203** of the stent **200.** In other embodiments, there can be more or fewer coating regions. Also, in other embodiments, the coating regions can have different number and types of coating layers than what are shown in the figures. Furthermore, the sidewall **203** can include openings (not shown) that extend through the inner **204** and outer **202** surfaces. Moreover, although the coating regions are shown to span an entire circumference of the stent, in some embodiments, less than the entire circumference may be covered by a coating region.

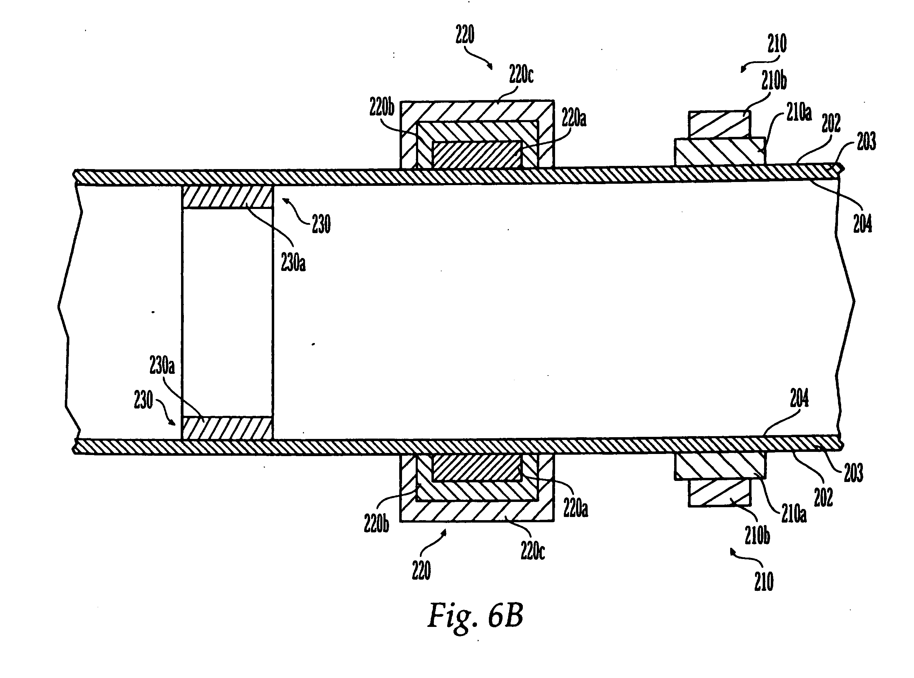

The first coating region **210** comprises two coating layers: a first coating layer **210a** and a first additional coating layer **210b.** As shown in the cut-away portion of **Fig. 6A** and the cross-sectional view of **Fig. 6B****,** the first additional layer **210b** does not cover the underlying first coating layer **210a.** In other embodiments, the first additional coating layer 210a can cover the entire first coating layer **210a** or if the first coating region includes other coating layers, those other coating layers.

The second coating region **220** comprises three coating layers in this embodiment; a second coating layer **220a,** a second additional coating layer **220b** and a third additional coating layer **220c.** In this embodiment, the third additional coating layer **220c** covers the entire underlying second additional coating layer **220b,** which covers the entire underlying second coating layer **220a.** (See cut-away in **Fig. 6A** and the cross-sectional view of **Fig. 6B****).** In other embodiments, the overlying coating layer may cover less than all of an underlying coating layer. Also in other embodiments the third coating region **230** can include more or fewer coating layers.

The third coating region **230** comprises a third coating layer **230a.** In this embodiment, the third coating layer **230a,** is not covered by any other coating layer. In other embodiments it may be covered by other coating layer(s).

In some embodiments, the first, second and/or third coating layers **210a, 220a,** and **230a** comprise a therapeutic agent. In certain embodiments, the first, second and/or third additional coating layers **220b, 220c** and **210b** comprise a biodegradable material and/or a therapeutic agent.

The various coating layers of the coating regions can be formed by applying a coating composition to the medical device. Coating compositions may be applied by any method to a surface of a stent or medical device to form a coating layer. Examples of suitable methods include, but are not limited to, spraying such as by conventional nozzle or ultrasonic nozzle, dipping, rolling, electrostatic deposition, and a batch process such as air suspension, pancoating or ultrasonic mist spraying. Other coating methods include screen printing, positive displacement coating (*i.e*., inkjet-printing technology), and spray coating combined with masking. Also, more than one coating method may be used.

Coating compositions for forming coating layers may include a polymeric material. The polymeric material should be a material that is biocompatible and avoids irritation to body tissue. Preferably the polymeric materials used in the coating composition of the present invention are selected from the following: polyurethanes, silicones (*e.g*., polysiloxanes and substituted polysiloxanes), and polyesters. Also preferable as a polymeric material are styrene-isobutylene-styrene copolymers. Other polymers that may be used include ones that may be dissolved and cured or polymerized on the stent or polymers having relatively low melting points that can be blended with biologically active materials. Additional suitable polymers include thermoplastic elastomers in general, polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate, copolymers of vinyl monomers, copolymers of vinyl monomers and olefins such as ethylene-methyl methacrylate copolymers, acrylonitrile-styrene copolymers, ABS (acrylonitrile-butadiene-styrene) resins, ethylene-vinyl acetate copolymers, polyamides such as Nylon 66 and polycaprolactone, alkyd resins, polycarbonates, polyoxymethylenes, polyimides, polyethers, epoxy resins, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM (ethylene-propylene-diene) rubbers, fluorosilicones, polyethylene glycol, polysaccharides, phospholipids, and combinations of the foregoing.

Preferably, for medical devices which undergo mechanical challenges, *e.g*., expansion and contraction, polymeric materials should be selected from elastomeric polymers such as silicones (*e.g*., polysiloxanes and substituted polysiloxanes), polyurethanes, thermoplastic elastomers, ethylene vinyl acetate copolymers, polyolefin elastomers, and EPDM rubbers. Because of the elastic nature of these polymers, the coating composition is capable of undergoing deformation under the yield point when the stent is subjected to forces, stress or mechanical challenge.

The biodegradable polymers that can be used to form the biodegradable coating layers include without limitation poly-L-lactic acid, poly-glycolic acid, polycaprolactone, poly(lactide-co-glycolide), poly(hydroxybutyrate), poly(hydroxybutyrate-co-valerate), polydioxanone, polyorthoester, polyanhydride, poly(D,L-lactic acid), poly(glycolic acid-co-trimethylene carbonate), polyphosphoester, polyphosphoester urethane, poly(amino acids), cyanoacrylates, poly(trimethylene carbonate), poly(iminocarbonate), copoly(etheresters)(e.g., PEO/PLA), polyalkylene oxalates, polyphosphazenes and biomolecules such as fibrin, fibrinogen, cellulose, starch, collagen, gel foam and hyaluronic acid.

The therapeutic agents that can be included in the coating layers include biologically active agents, and also genetic materials and pharmaceutical or nutraceutical materials. The genetic materials mean DNA or RNA, including, without limitation, of DNA/RNA encoding a useful protein stated below, intended to be inserted into a human body including viral vectors and non-viral vectors as well as anti-sense nucleic acid molecules such as DNA, RNA and RNAi. Viral vectors include adenoviruses, gutted adenoviruses, adeno-associated virus, retroviruses, alpha virus (Semliki Forest, Sindbis, *etc*.), lentiviruses, herpes simplex virus, ex vivo modified cells (*e.g*., stem cells, fibroblasts, myoblasts, satellite cells, pericytes, cardiomyocytes, skeletal myocytes, macrophage), replication competent viruses (*e.g*., ONYX-015), and hybrid vectors. Non-viral vectors include artificial chromosomes and mini-chromosomes, plasmid DNA vectors (*e.g*., pCOR), cationic polymers (*e.g*., polyethyleneimine, polyethyleneimine (PEI)) graft copolymers (*e.g*., polyether-PEI and polyethylene oxide-PEI), neutral polymers PVP, SP1017 (SUPRATEK), lipids or lipoplexes, nanoparticles and microparticles with and without targeting sequences such as the protein transduction domain (PTD). The biological materials include cells, yeasts, bacteria, proteins, peptides, cytokines and hormones. Examples for peptides and proteins include growth factors (FGF, FGF-1, FGF-2, VEGF, Endothelial Mitogenic Growth Factors, and epidermal growth factors, transforming growth factor and platelet derived endothelial growth factor, platelet derived growth factor, tumor necrosis factor, hepatocyte growth factor and insulin like growth factor), transcription factors, proteinkinases, CD inhibitors, thymidine kinase, and bone morphogenic proteins (BMP's), such as BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells may be of human origin (autologous or allogeneic) or from an animal source (xenogeneic), genetically engineered, if desired, to deliver proteins of interest at the transplant site. The delivery media can be formulated as needed to maintain cell function and viability. Cells include whole bone marrow, bone marrow derived mono-nuclear cells, progenitor cells (*e.g*., endothelial progentitor cells) stem cells (*e.g*., mesenchymal, hematopoietic, neuronal), pluripotent stem cells, fibroblasts, macrophage, and satellite cells.

Therapeutic agents also include non-genetic therapeutic agents, such as:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid, and mesalamine;
- antineoplastic/antiproliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxi and its analogs or derivatives;
- anesthetic agents such as lidocaine, bupivacaine, and ropivacaine;
- anti-coagulants such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory biologically active agent), dipyridamole, protamine, hirudin, prostaglandin inhibitors, platelet inhibitors, antiplatelet agents such as trapidil and liprostin, and tick antiplatelet peptides;
- DNA demethylating drugs such as 5-azacytidine, which is also categorized as a RNA or DNA metabolite that inhibit cell growth and induce apoptosis in certain cancer cells.
- vascular cell growth promotors such as growth factors, vascular Endothelial Growth Factors (FEGF, all types including VEGF-2), growth factor receptors, transcriptional activators, and translational promotors;
- vascular cell growth inhibitors such as antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents; vasodilating agents; and agents which interfere with endogenous vasoactive mechanisms;
- anti-oxidants, such as probucol;
- antibiotic agents, such as penicillin, cefoxitin, oxacillin, tobranycin, rapamycin (sirolimus);
- angiogenic substances, such as acidic and basic fibrobrast growth factors, estrogen including estradiol (E2), estriol (E3) and 17-Beta Estradiol; and
- biologically active agents for heart failure, such as digoxin, beta-blockers, angiotensin-converting enzyme (ACE) inhibitors including captopril and enalopril, statins and related compounds.

Preferred therapeutic agents include anti-proliferative drugs such as steroids, vitamins, and restenosis-inhibiting agents. Preferred restenosis-inhibiting agents include microtubule stabilizing agents such as Taxol, paclitaxel, paclitaxel analogues, derivatives, and mixtures thereof. For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt.

Other preferred therapeutic agents include nitroglycerin, nitrous oxides, nitric oxides, antibiotics, aspirins, digitalis, estrogen derivatives such as estradiol and glycosides.

Coating compositions suitable for applying therapeutic agents to the devices of the present invention preferably include a polymeric material and a therapeutic agent dispersed or dissolved in a solvent which does not alter or adversely impact the therapeutic properties of the biologically active material employed. Suitable polymers and therapeutics include, but are not limited to, those listed above.

Solvents used to prepare coating compositions include ones which can dissolve or suspend the polymeric material in solution. Examples of suitable solvents include, but are not limited to, tetrahydrofuran, methylethylketone, chloroform, toluene, acetone, isooctane, 1,1,1,-trichloroethane, dichloromethane, isopropanol, IPA, and mixtures thereof.

In another embodiment, the coating composition comprises a non-polymeric material. In another embodiment, the coating composition comprises entirely of a therapeutic agent. Coating compositions may be used to apply one type of therapeutic agent or a combination of therapeutic agents.

It should be appreciated that the features and components described herein may be used singly or in any combination thereof. Moreover, the present invention is not limited to only the embodiments specifically described herein, and may be used with medical devices other than stents. The disclosed system may be used to deliver a therapeutic agent to various types of body lumina, including but not limited to the esophagus, urinary tract, and intestines.

## Claims

1. A medical device for delivering a therapeutic agent to a body tissue of a patient comprising:
(a) a medical device having a surface;
(b) a first coating region (10) disposed on a first portion of the medical device surface, wherein the first coating region (10) comprises a first coating layer (10a) comprising a first therapeutic agent (9a);
(c) a second coating region (20) disposed on a second portion of the medical device surface, wherein the second coating region (20) comprises
(i) a second coating layer (20a) comprising a second therapeutic agent (9b); and
(ii) at least a first additional coating layer (20b) disposed over the second coating layer (20a), wherein the first additional coating layer (20b) comprises a first biodegradable or bioabsorbable material;
wherein the first coating layer (10a) is not covered by any other coating layer (20b);
and wherein the first additional coating layer (20b) prevents the second therapeutic agent (9b) of the second coating layer (20a) from beginning to release from the second coating layer (20a) at the same time as the first therapeutic agent (9a) of the first coating layer (10a) begins to release from the first coating layer (10a).

2. The medical device of claim 1, wherein the second therapeutic agent (9b) begins to release from the second coating layer (20a) when a significant part of the biodegradable first additional coating layer (20b) is degraded or absorbed.

3. The medical device of claim 1, wherein the first additional coating layer (20b) causes the therapeutic agents of the first coating layer (10a) and the second coating layer (20a) to be released over different time periods.

4. The medical device of claim 1, wherein the first therapeutic agent (9a) and the second therapeutic agent (9b) are the same.

5. The medical device of claim 1, wherein the first coating layer (10a) is not covered by any other coating layer.

6. The medical device of claim 1, wherein the first coating layer (10a) and the second coating layer (20a) are contiguous.

7. The medical device of claim 1, wherein the first additional coating layer (20b) is disposed directly over the second coating layer.

8. The medical device of claim 1, wherein the first additional coating layer (20b) is disposed indirectly over the second coating layer (20a).

9. The medical device of claim 8, further comprising an intermediate coating layer (20c) disposed between the second coating layer (20a) and the first additional coating layer (20b).

10. The medical device of claim 9, wherein the intermediate coating layer (20c) comprises a biodegradable or bioabsorbable material.

11. The medical device of claim 9, wherein the intermediate coating layer (20c) comprises a third therapeutic agent.

12. The medical device of claim 11, wherein the intermediate coating layer (20c) further comprises a biodegradable or bioabsorbable material.

13. The medical device of claim 1, wherein the first coating layer (10a) further comprises a polymeric material.

14. The medical device of claim 1, wherein the second coating layer (20a) further comprises a polymeric material.

15. The medical device of claim 1, wherein the first therapeutic agent (9a) comprises an anti-thrombogenic agent, an anti-angiogenesis agent, an anti-proliferative agent, a growth factor, or a radiochemical.

16. The medical device of claim 15, wherein the anti-proliferative agent comprises paclitaxel, a paclitaxel analogue or a paclitaxel derivative.

17. The medical device of claim 1, wherein the medical device is a stent having a tubular sidewall and wherein the first portion of the medical device surface lies along a circumference of the tubular sidewall.

18. The medical device of claim 1, wherein the medical device is a stent having a tubular sidewall and wherein the first portion of the medical device surface lies along a longitudinal axis of the tubular sidewall.

19. The medical device of claim 1, further comprising a third coating region (30) disposed over a third portion of the medical device surface wherein the third coating region (30) comprises
(i) a third coating layer (30a) comprising a third therapeutic agent (9c); and
(ii) at least a second additional coating layer (30b) disposed over the third coating layer (30a), wherein the second additional coating layer (30b) comprises a second biodegradable or bioabsorbable material.

20. The medical device of claim 19, wherein the first therapeutic agent (9a), the second therapeutic agent (9b) and the third therapeutic agent (9c) are the same.

21. The medical device of claim 19, wherein the second additional coating layer (30b) is disposed directly over the third coating layer (30a).

22. The medical device of claim 19, wherein the second additional coating layer (30b) is disposed indirectly over the third coating layer (30a).

23. The medical device of claim 22, further comprising an intermediate coating layer (20c) disposed between the second coating layer (20a) and the first additional coating layer (20b).

24. The medical device of claim 23, wherein the intermediate coating layer (20c) comprises a biodegradable or bioabsorbable material.

25. The medical device of claim 23, wherein the intermediate coating layer (20c) comprises a fourth therapeutic agent.

26. The medical device of claim 25, wherein the intermediate coating layer (20c) further comprises a biodegradable or bioabsorbable material.

27. The medical device of claim 19, wherein the first coating layer (10a), is contiguous with at least the second coating layer (20a) or the third coating layer (30a).

28. The medical device of claim 19, further comprising at least a third additional coating layer disposed over the second additional coating layer (30b).

29. The medical device of claim 28, wherein the third additional coating layer comprises a third biodegradable or bioabsorbable material.

30. The medical device of claim 19, wherein the first and second biodegradable or bioabsorbable materials degrade at the same rate.

31. The medical device of claim 19, wherein the first and second biodegradable or bioabsorbable materials degrade at different rates.

32. The medical device of claim 19, wherein the first additional coating layer (20b) has a first thickness and the second additional coating layer (30b) has a second thickness and wherein the first and second thicknesses are not the same.

33. The medical device of claim 1, wherein the medical device is a stent having a tubular sidewall having an inner surface and an outer surface and
wherein the first coating region (10) and second coating region (20) are disposed on the outer surface.

34. The medical device of claim 33, further comprising a third coating region disposed on the inner surface, wherein the third coating region comprises
(i) a third coating layer comprising a third therapeutic agent;
and
(ii) at least a second additional coating layer disposed over the third coating layer, wherein the second additional coating layer comprises a second biodegradable or bioabsorbable material.

35. The medical device of claim 1, wherein the medical device is a stent,
wherein the first coating layer (10a) further comprises a first polymeric material,
wherein the second coating layer (20a) further comprises a second polymeric material, and
wherein the first coating layer (10a) and the second coating layer (20a) are contiguous and wherein the first additional coating layer (20b) is disposed directly over the second coating layer (20a).

36. The stent of claim 35, wherein the second therapeutic agent (9b) begins to release from the second coating layer (20a) when a significant part of the biodegradable first additional coating layer (20b) is degraded or absorbed.

37. The stent of claim 35, wherein the first additional coating layer (20b) causes the therapeutic agents (9a) of the first coating layer (10a) and the therapeutic agent (9b) of the second coating layers (20a) to be released over different time periods.

38. The medical device of claim 1, wherein the medical device is a stent having a tubular sidewall and wherein the surface is an outer surface, further comprising a third coating region disposed on a portion of an inner surface of the tubular sidewall, the third coating region comprising
(i) a third coating layer comprising a third therapeutic agent and a polymeric material; and
(ii) at least a second additional coating layer disposed over the third coating layer, wherein the second additional coating layer comprises a second biodegradable or bioabsorbable material,
wherein the first coating layer (10a), and the second coating layer (20a) are contiguous, and wherein the first additional coating layer (20b) is disposed directly over the second coating layer (20a).

39. The stent of claim 38, wherein the second therapeutic agent (9b) begins to release from the second coating layer (20a) when a significant part of the biodegradable first additional coating layer (20b) is degraded or absorbed.

40. The stent of claim 38, wherein the first additional coating layer (20b) causes the therapeutic agents (9a) of the first coating layer (10a) and the therapeutic agent (9b) of the second coating layers (20a) to be released over different time periods.

41. The stent of claim 38, further comprising at least a third additional coating layer disposed over the second additional coating layer.

42. The stent of claim 41, wherein the third additional coating layer comprises a third biodegradable or bioabsorbable material.

43. The stent of claim 41 wherein the second additional coating layer further comprises a fourth therapeutic agent.

44. The stent of claim 42 wherein the third additional coating layer further comprises a fifth therapeutic agent.

## Patentansprüche

1. Medizinische Vorrichtung zur Zuführung eines therapeutischen Wirkstoffes an ein Körpergewebe von einem Patienten, umfassend:
(a) eine medizinische Vorrichtung, die eine Oberfläche aufweist;
(b) eine erste Beschichtungsregion (10), angeordnet auf einem ersten Teil der Oberfläche der medizinischen Vorrichtung, wobei die erste Beschichtungsregion (10) eine erste Beschichtungsschicht (10a) umfasst, die einen ersten therapeutischen Wirkstoff (9a) umfasst;
(c) eine zweite Beschichtungsregion (20), angeordnet auf einem zweiten Teil von der Oberfläche der medizinischen Vorrichtung,
wobei die zweite Beschichtungsregion (20) umfasst:
(i) eine zweite Beschichtungsschicht (20a), umfassend einen zweiten therapeutischen Wirkstoff (9b); und
(ii) mindestens eine erste zusätzliche Beschichtungsschicht (20b), angeordnet über der zweiten Beschichtungsschicht (20a), wobei die erste zusätzliche Beschichtungsschicht (20b) ein erstes biodegradierbares oder bioabsorbierbares Material umfasst;
wobei die erste Beschichtungsschicht (10a) nicht von irgendeiner anderen Beschichtungsschicht (20b) abgedeckt ist;
und wobei die erste zusätzliche Beschichtungsschicht (20b) den zweiten therapeutischen Wirkstoff (9b) der zweiten Beschichtungsschicht (20a) davon abhält, zu beginnen, von der zweiten Beschichtungsschicht (20a) abgegeben zu werden, zur selben Zeit, wie der erste therapeutische Wirkstoff (9a) der ersten Beschichtungsschicht (10a) beginnt, von der ersten Beschichtungsschicht (10a) abgegeben zu werden.

2. Medizinische Vorrichtung nach Anspruch 1, wobei der zweite therapeutische Wirkstoff (9b) beginnt, von der zweiten Beschichtungsschicht (20a) abgegeben zu werden, wenn ein signifikanter Anteil der biodegradierbaren ersten zusätzlichen Beschichtungsschicht (20b) degradiert oder absorbiert ist.

3. Medizinische Vorrichtung nach Anspruch 1, wobei die erste zusätzliche Beschichtungsschicht (20b) bewirkt, dass die therapeutischen Wirkstoffe der ersten Beschichtungsschicht (10a) und der zweiten Beschichtungsschicht (20a) über unterschiedliche Zeitperioden abgegeben werden.

4. Medizinische Vorrichtung nach Anspruch 1, wobei der erste therapeutische Wirkstoff (9a) und der zweite therapeutische Wirkstoff (9b) gleich sind.

5. Medizinische Vorrichtung nach Anspruch 1, wobei die erste Beschichtungsschicht (10a) nicht von irgendeiner anderen Beschichtungsschicht abgedeckt ist.

6. Medizinische Vorrichtung nach Anspruch 1, wobei die erste Beschichtungsschicht (10a) und die zweite Beschichtungsschicht (20a) zusammenhängend sind.

7. Medizinische Vorrichtung nach Anspruch 1, wobei die erste zusätzliche Beschichtungsschicht (20b) direkt über der zweiten Beschichtungsschicht angeordnet ist.

8. Medizinische Vorrichtung nach Anspruch 1, wobei die erste zusätzliche Beschichtungsschicht (20b) indirekt über der zweiten Beschichtungsschicht (20a) angeordnet ist.

9. Medizinische Vorrichtung nach Anspruch 8, weiter umfassend eine Zwischenbeschichtungsschicht (20c), angeordnet zwischen der zweiten Beschichtungsschicht (20a) und der ersten zusätzlichen Beschichtungsschicht (20b).

10. Medizinische Vorrichtung nach Anspruch 9, wobei die Zwischenbeschichtungsschicht (20c) ein biodegradierbares oder bioabsorbierbares Material umfasst.

11. Medizinische Vorrichtung nach Anspruch 9, wobei die Zwischenbeschichtungsschicht (20c) einen dritten therapeutischen Wirkstoff umfasst.

12. Medizinische Vorrichtung nach Anspruch 11, wobei die Zwischenbeschichtungsschicht (20c) weiter ein biodegradierbares oder bioabsorbierbares Material umfasst.

13. Medizinische Vorrichtung nach Anspruch 1, wobei die erste Beschichtungsschicht (10a) weiter ein polymeres Material umfasst.

14. Medizinische Vorrichtung nach Anspruch 1, wobei die zweite Beschichtungsschicht (20a) weiter ein polymeres Material umfasst.

15. Medizinische Vorrichtung nach Anspruch 1, wobei der erste therapeutische Wirkstoff (9a) einen antithrombogenen Wirkstoff, einen Antiangiogenesewirkstoff, einen antiproliferativen Wirkstoff, einen Wachstumsfaktor oder eine Radiochemikalie umfasst.

16. Medizinische Vorrichtung nach Anspruch 15, wobei der antiproliferative Wirkstoff Paclitaxel, ein Paclitaxelanalog, oder ein Paclitaxelderivativ umfasst.

17. Medizinische Vorrichtung nach Anspruch 1, wobei die medizinische Vorrichtung ein Stent ist, der eine röhrenförmige Seitenwand aufweist und
wobei der erste Teil der Oberfläche der medizinischen Vorrichtung entlang eines Umfangs der röhrenförmigen Seitenwand liegt.

18. Medizinische Vorrichtung nach Anspruch 1, wobei die medizinische Vorrichtung ein Stent ist, der eine röhrenförmige Seitenwand aufweist und
wobei der erste Teil der Oberfläche der medizinischen Vorrichtung entlang einer Längsachse der röhrenförmigen Seitenwand liegt.

19. Medizinische Vorrichtung nach Anspruch 1, weiter umfassend eine dritte Beschichtungsregion (30), angeordnet über einem dritten Teil der Oberfläche der medizinischen Vorrichtung, wobei die dritte Beschichtungsregion (30) umfasst
(i) eine dritte Beschichtungsschicht (30a), umfassend einen dritten therapeutischen Wirkstoff (9c); und
(ii) mindestens eine zweite zusätzliche Beschichtungsschicht (30b), angeordnet über der dritten Beschichtungsschicht (30a), wobei die zweite zusätzliche Beschichtungsschicht (30b) ein zweites biodegradierbares oder bioabsorbierbares Material umfasst.

20. Medizinische Vorrichtung nach Anspruch 19, wobei der erste therapeutische Wirkstoff (9a), der zweite therapeutische Wirkstoff (9b) und der dritte therapeutische Wirkstoff (9c) gleich sind.

21. Medizinische Vorrichtung nach Anspruch 19, wobei die zweite zusätzliche Beschichtungsschicht (30b) direkt über der dritten Beschichtungsschicht (30a) angeordnet ist.

22. Medizinische Vorrichtung nach Anspruch 19, wobei die zweite zusätzliche Beschichtungsschicht (30b) indirekt über der dritten Beschichtungsschicht (30a) angeordnet ist.

23. Medizinische Vorrichtung nach Anspruch 22, weiter umfassend eine Zwischenbeschichtungsschicht (20c), angeordnet zwischen der zweiten Beschichtungsschicht (20a) und der ersten zusätzlichen Beschichtungsschicht (20b).

24. Medizinische Vorrichtung nach Anspruch 23, wobei die Zwischenbeschichtungsschicht (20c) ein biodegradierbares oder bioabsorbierbares Material umfasst.

25. Medizinische Vorrichtung nach Anspruch 23, wobei die Zwischenbeschichtungsschicht (20c) einen vierten therapeutischen Wirkstoff umfasst.

26. Medizinische Vorrichtung nach Anspruch 25, wobei die Zwischenbeschichtungsschicht (20c) weiter ein biodegradierbares oder bioabsorbierbares Material umfasst.

27. Medizinische Vorrichtung nach Anspruch 19, wobei die erste Beschichtungsschicht (10a) mindestens mit der zweiten Beschichtungsschicht (20a) oder der dritten Beschichtungsschicht (30a) zusammenhängend ist.

28. Medizinische Vorrichtung nach Anspruch 19, weiter umfassend mindestens eine dritte zusätzliche Beschichtungsschicht, angeordnet über der zweiten zusätzlichen Beschichtungsschicht (30b).

29. Medizinische Vorrichtung nach Anspruch 28, wobei die dritte zusätzliche Beschichtungsschicht ein drittes biodegradierbares oder bioabsorbierbares Material umfasst.

30. Medizinische Vorrichtung nach Anspruch 19, wobei die ersten und zweiten biodegradierbaren oder bioabsorbierbaren Materialien mit derselben Rate degradieren.

31. Medizinische Vorrichtung nach Anspruch 19, wobei die ersten und zweiten biodegradierbaren oder bioabsorbierbaren Materialien mit unterschiedlichen Raten degradieren.

32. Medizinische Vorrichtung nach Anspruch 19, wobei die erste zusätzliche Beschichtungsschicht (20b) eine erste Dicke aufweist und die zweite zusätzliche Beschichtungsschicht (30b) eine zweite Dicke aufweist, und
wobei die ersten und zweiten Dicken nicht gleich sind.

33. Medizinische Vorrichtung nach Anspruch 1, wobei die medizinische Vorrichtung ein Stent ist, der eine röhrenförmige Seitenwand aufweist, die eine innere Oberfläche und eine äußere Oberfläche hat, und wobei die erste Beschichtungsregion (10) und zweite Beschichtungsregion (20) auf der äußeren Oberfläche angeordnet sind.

34. Medizinische Vorrichtung nach Anspruch 33, weiter umfassend eine dritte Beschichtungsregion angeordnet auf der inneren Oberfläche, wobei die dritte Beschichtungsregion umfasst
(i) eine dritte Beschichtungsschicht umfassend einen dritten therapeutischen Wirkstoff; und
(ii) mindestens eine zweite zusätzliche Beschichtungsschicht angeordnet über der dritten Beschichtungsschicht, wobei die zweite zusätzliche Beschichtungsschicht ein zweites biodegradierbares oder bioabsorbierbares Material umfasst.

35. Medizinische Vorrichtung nach Anspruch 1, wobei die medizinische Vorrichtung ein Stent ist,
wobei die erste Beschichtungsschicht (10a) weiter ein erstes polymeres Material umfasst,
wobei die zweite Beschichtungsschicht (20a) weiter ein zweites polymeres Material umfasst, und
wobei die erste Beschichtungsschicht (10a) und die zweite Beschichtungsschicht (20a) zusammenhängend sind, und wobei die erste zusätzliche Beschichtungsschicht (20b) direkt über der zweiten Beschichtungsschicht (20a) angeordnet ist.

36. Stent nach Anspruch 35, wobei der zweite therapeutische Wirkstoff (9b) beginnt, von der zweiten Beschichtungsschicht (20a) abgegeben zu werden, wenn ein signifikanter Anteil der biodegradierbaren ersten zusätzlichen Beschichtungsschicht (20b) degradiert oder absorbiert ist.

37. Stent nach Anspruch 35, wobei die erste zusätzliche Beschichtungsschicht (20b) bewirkt, dass der therapeutische Wirkstoff (9a) der ersten Beschichtungsschicht (10a) und der therapeutische Wirkstoff (9b) der zweiten Beschichtungsschicht (20a) über unterschiedliche Zeitperioden abgegeben werden.

38. Medizinische Vorrichtung nach Anspruch 1, wobei die medizinische Vorrichtung ein Stent ist, der eine röhrenförmige Seitenwand aufweist und
wobei die Oberfläche eine äußere Oberfläche ist, weiter umfassend eine dritte Beschichtungsregion, angeordnet auf einem Teil einer inneren Oberfläche der röhrenförmigen Seitenwand, wobei die dritte Beschichtungsregion umfasst
(i) eine dritte Beschichtungsschicht umfassend einen dritten therapeutischen Wirkstoff und ein polymeres Material; und
(ii) mindestens eine zweite zusätzliche Beschichtungsschicht angeordnet über der dritten Beschichtungsschicht, wobei die zweite zusätzliche Beschichtungsschicht ein zweites biodegradierbares oder bioabsorbierbares Material umfasst,
wobei die erste Beschichtungsschicht (10a) und die zweite Beschichtungsschicht (20a) zusammenhängend sind, und wobei die erste zusätzliche Beschichtungsschicht (20b) direkt über der zweiten Beschichtungsschicht (20a) angeordnet ist.

39. Stent nach Anspruch 38, wobei der zweite therapeutische Wirkstoff (9b) beginnt, von der zweiten Beschichtungsschicht (20a) abgegeben zu werden, wenn ein signifikanter Anteil der biodegradierbaren ersten zusätzlichen Beschichtungsschicht (20b) degradiert oder absorbiert ist.

40. Stent nach Anspruch 38, wobei die erste zusätzliche Beschichtungsschicht (20b) bewirkt, dass der therapeutische Wirkstoff (9a) der ersten Beschichtungsschicht (10a) und der therapeutische Wirkstoff (9b) der zweiten Beschichtungsschicht (20a) über unterschiedliche Zeitperioden abgegeben werden.

41. Stent nach Anspruch 38, weiter umfassend mindestens eine dritte zusätzliche Beschichtungsschicht, angeordnet über der zweiten zusätzlichen Beschichtungsschicht.

42. Stent nach Anspruch 41, wobei die dritte zusätzliche Beschichtungsschicht ein drittes biodegradierbares oder bioabsorbierbares Material umfasst.

43. Stent nach Anspruch 41, wobei die zweite zusätzliche Beschichtungsschicht weiter einen vierten therapeutischen Wirkstoff umfasst.

44. Stent nach Anspruch 42, wobei die dritte zusätzliche Beschichtungsschicht weiter einen fünften therapeutischen Wirkstoff umfasst.

## Revendications

1. Dispositif médical pour administrer un agent thérapeutique à un tissu corporel d'un patient, comprenant :
(a) un dispositif médical ayant une surface ;
(b) une première région revêtue (10) disposée sur une première portion de la surface du dispositif médical, ladite première région revêtue (10) comprenant une première couche de revêtement (10a) comprenant un premier agent thérapeutique (9a) ;
(c) une seconde région revêtue (20) disposée sur une seconde portion de la surface du dispositif médical, ladite seconde région revêtue (20) comprenant
(i) une seconde couche de revêtement (20a) comprenant un second agent thérapeutique (9b) ; et
(ii) au moins une première couche de revêtement additionnelle (20b) disposée par-dessus la seconde couche de revêtement (20a), ladite première couche de revêtement additionnelle (20b) comprenant un premier matériau biodégradable ou bioabsorbable ;
dans lequel la première couche de revêtement (10a) n'est couverte par aucune autre couche de revêtement (20b) ;
et dans lequel la première couche de revêtement additionnelle (20b) empêche que le second agent thérapeutique (9b) de la seconde couche de revêtement (20a) commence à se dégager hors de la seconde couche de revêtement (20a) en même temps que le premier agent thérapeutique (9a) de la première couche de revêtement (10a) commence à se dégager hors de la première couche de revêtement (10a).

2. Dispositif médical selon la revendication 1, dans lequel le second agent thérapeutique (9b) commence à se dégager hors de la seconde couche de revêtement (20a) quand une partie significative de la première couche de revêtement additionnelle biodégradable (20b) est dégradée ou absorbée.

3. Dispositif médical selon la revendication 1, dans lequel la première couche de revêtement additionnelle (20b) amène les agents thérapeutiques de la première couche de revêtement (10a) et de la seconde couche de revêtement (20a) à se dégager sur des périodes temporelles différentes.

4. Dispositif médical selon la revendication 1, dans lequel le premier agent thérapeutique (9a) et le second agent thérapeutique (9b) sont le même.

5. Dispositif médical selon la revendication 1, dans lequel la première couche de revêtement (10a) n'est couverte par aucune autre couche de revêtement.

6. Dispositif médical selon la revendication 1, dans lequel la première couche de revêtement (10a) et la seconde couche de revêtement (20a) sont contiguës.

7. Dispositif médical selon la revendication 1, dans lequel la première couche de revêtement additionnelle (20b) est disposée directement par-dessus la seconde couche de revêtement.

8. Dispositif médical selon la revendication 1, dans lequel la première couche de revêtement additionnelle (20b) est disposée indirectement par-dessus la seconde couche de revêtement (20a).

9. Dispositif médical selon la revendication 8, comprenant en outre une couche de revêtement intermédiaire (20c) disposée entre la seconde couche de revêtement (20a) et la première couche de revêtement additionnelle (20b).

10. Dispositif médical selon la revendication 9, dans lequel la couche de revêtement intermédiaire (20c) comprend un matériau biodégradable ou bioabsorbable.

11. Dispositif médical selon la revendication 9, dans lequel la couche de revêtement intermédiaire (20c) comprend un troisième agent thérapeutique.

12. Dispositif médical selon la revendication 11, dans lequel la couche de revêtement intermédiaire (20c) comprend en outre un matériau biodégradable ou bioabsorbable.

13. Dispositif médical selon la revendication 1, dans lequel la première couche de revêtement (10a) comprend en outre un matériau polymère.

14. Dispositif médical selon la revendication 1, dans lequel la seconde couche de revêtement (20a) comprend en outre un matériau polymère.

15. Dispositif médical selon la revendication 1, dans lequel le premier agent thérapeutique (9a) comprend un agent anti-thrombose, un agent anti-angiogénèse, un agent anti-prolifération, un facteur de croissance, ou un produit radiochimique.

16. Dispositif médical selon la revendication 15, dans lequel l'agent anti-prolifération comprend du paclitaxel, un produit analogue au paclitaxel, ou un dérivé de paclitaxel.

17. Dispositif médical selon la revendication 1, dans lequel le dispositif médical est un stent ayant une paroi latérale tubulaire, et dans lequel la première portion de la surface du dispositif médical est située le long d'une circonférence de la paroi latérale tubulaire.

18. Dispositif médical selon la revendication 1, dans lequel le dispositif médical est un stent ayant une paroi latérale tubulaire, et dans lequel la première portion de la surface du dispositif médical est située le long d'un axe longitudinal de la paroi latérale tubulaire.

19. Dispositif médical selon la revendication 1, comprenant en outre une troisième région de revêtement (30) disposée par-dessus une troisième portion de la surface du dispositif médical, dans lequel la troisième région de revêtement (30) comprend
(i) une troisième couche de revêtement (30a) comprenant un troisième agent thérapeutique (9c) ; et
(ii) au moins une seconde couche de revêtement additionnelle (30b) disposée par-dessus la troisième couche de revêtement (30a), dans lequel la seconde couche de revêtement additionnelle (30b) comprend un matériau biodégradable ou bioabsorbable.

20. Dispositif médical selon la revendication 19, dans lequel le premier agent thérapeutique (9a), le second agent thérapeutique (9b) et le troisième agent thérapeutique (9c) sont le même.

21. Dispositif médical selon la revendication 19, dans lequel la seconde couche de revêtement additionnel (30b) est disposée directement par-dessus la troisième couche de revêtement (30a).

22. Dispositif médical selon la revendication 19, dans lequel la seconde couche de revêtement additionnel (30b) est disposée indirectement par-dessus la troisième couche de revêtement (30a).

23. Dispositif médical selon la revendication 22, comprenant en outre une couche de revêtement intermédiaire (20c) disposée entre la seconde couche de revêtement (20a) et la première couche de revêtement additionnelle (20b).

24. Dispositif médical selon la revendication 23, dans lequel la couche de revêtement intermédiaire (20c) comprend un matériau biodégradable ou bioabsorbable.

25. Dispositif médical selon la revendication 23, dans lequel la couche de revêtement intermédiaire (20c) comprend un quatrième agent thérapeutique.

26. Dispositif médical selon la revendication 25, dans lequel la couche de revêtement intermédiaire (20c) comprend en outre un matériau biodégradable ou bioabsorbable.

27. Dispositif médical selon la revendication 19, dans lequel la première couche de revêtement (10a) est contiguë au moins à la seconde couche de revêtement (20a) ou à la troisième couche de revêtement (30a).

28. Dispositif médical selon la revendication 19, comprenant en outre au moins une troisième couche de revêtement additionnelle disposée par-dessus la seconde couche de revêtement additionnelle (30b).

29. Dispositif médical selon la revendication 28, dans lequel la troisième couche de revêtement additionnelle comprend un troisième matériau biodégradable ou bioabsorbable.

30. Dispositif médical selon la revendication 19, dans lequel le premier et le second matériau biodégradable ou bioabsorbables se

31. Dispositif médical selon la revendication 19, dans lequel le premier et le second matériau biodégradable ou bioabsorbables se dégradent à des vitesses différentes.

32. Dispositif médical selon la revendication 19, dans lequel la première couche de revêtement additionnelle (20b) a une première épaisseur et la seconde couche de revêtement additionnelle (30b) a une seconde épaisseur, et dans lequel la première épaisseur et la seconde épaisseur ne sont pas la même.

33. Dispositif médical selon la revendication 1, dans lequel le dispositif médical est un stent ayant une paroi latérale tubulaire ayant une surface intérieure et une surface extérieure, et dans lequel la première région revêtue (10) et la seconde région revêtue (20) sont disposées sur la surface extérieure.

34. Dispositif médical selon la revendication 33, comprenant en outre une troisième région revêtue disposée sur la surface intérieure, dans lequel la troisième région revêtue comprend
(i) une troisième couche de revêtement comprenant un troisième agent thérapeutique ; et
(ii) au moins une seconde couche de revêtement additionnelle disposée par-dessus la troisième couche de revêtement, dans lequel la seconde couche de revêtement additionnelle comprend un second matériau biodégradable ou bioabsorbable.

35. Dispositif médical selon la revendication 1, le 10 dispositif médical étant un stent
dans lequel la première couche de revêtement (10a) comprend un premier matériau polymère,
dans lequel la seconde couche de revêtement (20a) comprend en outre un second matériau polymère, et
dans lequel la première couche de revêtement (10a) et la seconde couche de revêtement (20a) sont contiguës, et dans lequel la première couche de revêtement (20b) est disposée directement par-dessus la seconde couche de revêtement (20a).

36. Stent selon la revendication 35, dans lequel le second agent thérapeutique (9b) commence à se dégager hors de la seconde couche de revêtement (20a) quand une partie significative de la première couche de revêtement additionnel biodégradable (20b) est dégradée ou absorbée.

37. Stent selon la revendication 35, dans lequel la première couche de revêtement additionnel (20b) amène l'agent thérapeutique (9a) de la première couche de revêtement (10a) et l'agent thérapeutique (9b) de la seconde couche de revêtement (20a) à se dégager sur des périodes temporelles différentes.

38. Dispositif médical selon la revendication 1, dans lequel le dispositif médical est un stent ayant une paroi latérale tubulaire, et dans lequel la surface est une surface extérieure, comprenant en outre une troisième région revêtue disposée sur une portion d'une surface intérieure de la paroi latérale tubulaire, la troisième région revêtue comprenant
(i) une troisième couche de revêtement comprenant un troisième agent thérapeutique et un matériau polymère ; et
(ii) au moins une seconde couche de revêtement additionnelle disposée par-dessus la troisième couche de revêtement, dans lequel la seconde couche de revêtement additionnelle comprend un second matériau biodégradable ou bioabsorbable,
dans lequel la première couche de revêtement (10a) et la seconde couche de revêtement (20a) sont contiguës, et dans lequel la première couche de revêtement additionnelle (20b) est disposée directement par-dessus la seconde couche de revêtement (20a).

39. Stent selon la revendication 38, dans lequel le second agent thérapeutique (9b) commence à se dégager hors de la seconde couche de revêtement (20a) quand une partie significative de la première couche de revêtement additionnelle biodégradable (20b) est dégradée ou absorbée.

40. Stent selon la revendication 38, dans lequel la première couche de revêtement additionnelle (20b) amène l'agent thérapeutique (9a) de la première couche de revêtement (10a) et l'agent thérapeutique (9b) de la seconde couche de revêtement (20a) à se dégager sur des périodes temporelles différentes.

41. Stent selon la revendication 38, comprenant en outre au moins une troisième couche de revêtement additionnelle disposée par-dessus la seconde couche de revêtement additionnel.

42. Stent selon la revendication 41, dans lequel la troisième couche de revêtement additionnelle comprend un troisième matériau biodégradable ou bioabsorbable.

43. Stent selon la revendication 41, dans lequel la seconde couche de revêtement additionnelle comprend en outre un quatrième agent thérapeutique.

44. Stent selon la revendication 42, dans lequel la troisième couche de revêtement additionnelle comprend en outre un cinquième agent thérapeutique.
